# FASCICULE DE BREVET EUROPEEN

(11) **EP 0 689 401 B1**
(45) Date de publication et mention de la délivrance du brevet: **07.07.1999**
(21) Numéro de dépôt: 94906241.8
(22) Date de dépôt: 03.02.1994
(51) Int. Cl.: A61B 17/58

(54) **DISPOSITIF GLOBAL DE STABILISATION DU RACHIS**
UNIVERSALER STABILISATOR FÜR DIE WIRBELSÄULE
GLOBAL VERTEBRAL FIXATION DEVICE

(30) Priorité: 18.01.1994 FR 9400457
(43) Date de publication de la demande: 03.01.1996
(73) Titulaire: SCIENCE ET MEDECINE (Société anonyme), 75014 Paris (FR); Bréard, Francis Henri, 75014 Paris (FR)
(72) Inventeur: BREARD, Francis, Henri, F-75014 Paris (FR)
(86) Numéro de dépôt international: FR9400130
(87) Numéro de publication internationale: WO9519149

(56) Documents cités:
- EP-A- 0 441 084
- FR-A- 2 672 202
- FR-A- 2 674 118
- FR-A- 2 694 182

## Description

La présente invention se rapporte à un dispositif de stabilisation de la colonne vertébrale.
- Depuis Harrington en 1965, il est connu de traiter les déformations de la colonne vertébrale par des plaques ou barres métalliques percées et fixées le long du segment affecté du rachis au moyen de vis ou d'agrafes.

Deux problèmes se posent :
1. L'utilisation de ces plaques ou barres métalliques entraîne une solllication importante au niveau des vertèbres immédiates sus et sous-jacentes non endommagées, ce qui provoque à moyen terme leur instabilité, étant donné que toute articulation le long du segment affecté du rachis est bloquée.
2. Pour réaliser l'ostéosynthèse, le problème principal est celui du siège de l'implantation des vis ; le pédicule constitue une zone peu connue et délicate du rachis pour les chirurgiens-orthopédistes. Pour implanter correctement une vis dans un pédicule, il faut être absolument certain du point d'entrée et de la direction du forage.
   Le positionnement de l'implant dans la vertèbre doit se faire selon l'axe anatomique du pédicule avec une inclinaison variable d'une vertèbre à l'autre de l'ordre de 15° par rapport au plan sagital médian, tout en assurant l'alignement des vis pour la fixation de la plaque. En effet, une des conditions indispensables. pour diminuer les contraintes en torsion sur chaque vis et rendre le verrouillage effectif, est que la plaque doit être parfaitement tangente au niveau de chaque pédicule.

- Il est également connu d'utiliser un système de stabilisation souple (brevets FR-A-2 642 645 et FR-A-2 672 202) permettant de compenser l'instabilité en autorisant un débattement des vertèbres suffisant pour ne pas gêner le patient dans ses mouvements de flexion ou de torsion du tronc. Mais cette méthode, bien que résolvant certains des problèmes ci-dessus mentionnés, ne peut être utilisée pour les cas graves où l'arthrodèse est nécessaire.

La présente invention se propose de remédier à ces inconvénients, et pour ce faire, elle a pour objet un dispositif global de stabilisation du rachis permettant :
- la combinaison des systèmes souple et rigide,
- la substitution d'un système rigide à un système souple déjà mis en place en première intention, et vice-versa, sans qu'il y ait à explanter l'implant,
- la mise en place d'un système souple dans le prolongement d'un montage d'arthrodèse.

Ce dispositif global de stabilisation du rachis, destiné à être mis en place entre au moins deux vertèbres consécutives, comprend des implants pédiculaires implantables chacun dans une vertèbre respective, chaque implant associé à différents types d'entretoises interchangeables, aptes à recevoir un ou plusieurs plaques, barres rigides ou liens souples, à titre précaire ou définitif.

Pour permettre une interchangeabilité des éléments souples/rigides au niveau de chaque vertèbre, chaque élément est de préférence distinct d'une vertèbre à l'autre.

Chaque implant est formé d'une tige intra-osseuse suivie d'un axe extra-osseux par lequel se fera l'assemblage avec un type d'entretoise. Cet axe est de préférence fileté en son extrémité supérieure et lisse à sa base.

I - Selon un premier mode préférentiel de l'invention, l'entretoise est percée de part en part et constituée d'un segment de sphère seul, ou d'un segment de sphère prolongé d'un segment cylindrique, le segment de sphère étant destiné à recevoir une plaque rigide, le segment cylindrique étant destiné à recevoir un lien souple.

Le chirurgien-orthopédiste dispose d'un système de stabilisation souple qui pourra être mis en place au moins à l'une des deux extrémités d'un montage d'arthrodèse, évitant ainsi d'endommager les autres vertèbres.
1) Selon une première possibilité, cette entretoise reçoit un premier type de plaque : la plaque comporte, au moins en chacune de ses extrémités, une concavité parfaitement congruente à la convexité du segment de sphère et suivie d'un perçage débouchant sur l'autre face dans une concavité oblongue suivant l'axe longitudinal de la plaque.
   Avec ce type de plaque, l'implant reçoit également un segment de sphère présentant de préférence une même convexité sphérique que celle du segment de sphère précédent, comme il sera décrit plus en détail, ci-après, dans les modes de réalisation du dispositif global conforme à l'invention.
   Cet assemblage permet un débattement omnidirectionnel de la plaque par rapport à l'implant lors de sa mise en place tout en conservant par la suite une fixation rigide. Ainsi le chirurgien-orthopédiste pourra placer l'implant dans la position désirée, puis fixer aisément la plaque rigide, chaque plaque étant, bien entendu, fixée par au moins deux implants consécutifs.
2) Selon une deuxième possibilité, cette entretoise reçoit un deuxième type de plaque : la plaque comporte, au moins à chacune de ses extrémités, une concavité parfaitement congruente à la convexité du segment de sphère, et suivie d'un perçage débouchant sur l'autre face. L'implant reçoit avec ce type de plaque :
   . une rondelle comportant une concavité congruente à la convexité d'un segment de sphère et suivie d'un perçage débouchant sur l'autre face,
   . un segment de sphère percé de part en part et congruent à la concavité de la rondelle.

   Il va de soi que la plaque et la rondelle, comportant une concavité identique, peuvent être inversées, étant précisé que les segments de sphère sont similaires les uns aux autres.
   Cet assemblage permet un débattement omnidirectionnel de l'ordre de 15° de la plaque par rapport à l'implant, lors de sa mise en place. En fonction de la position désirée de l'implant, la rondelle glissera aux extrémités du perçage de la plaque jusqu'à ce qu'elle puisse se stabiliser.
3) Selon une troisième possibilité, l'implant est associé à une, deux ou même plusieurs plaques de même type que celles décrites précédemment.
   Dans le cas d'une association avec deux plaques, l'entretoise est uniquement constituée du segment de sphère.
   - Avec le premier type de plaque, il suffira d'insérer entre la plaque et le segment de sphère, une autre plaque et un autre segment de sphère, la concavité sphérique de la plaque recevant la convexité sphérique de celui-ci.
   - Avec le deuxième type de plaque, il suffira d'insérer entre la rondelle et un segment de sphère, un autre segment de sphère, et une autre plaque et rondelle, comme il sera décrit plus en détail, ci-après, dans les modes de réalisation du dispositif global conforme à l'invention.

Le débattement latéral de l'implant se fera alors par rapport à chacune des plaques, lors de leur mise en place.

Ainsi plusieurs plaques peuvent être mises en place, bloquant l'articulation le long du segment affecté.

Un système d'arthrodèse, de préférence distinct au niveau de chaque articulation, permet la substitution d'un système rigide à un système souple déjà mis en place en première intention, et vice-versa, au niveau de chaque articulation.

Il conviendra également de réaliser une entretoise constituée uniquement du segment cylindrique, destiné à recevoir un lien souple, ou d'un segment cylindrique comprenant en son milieu un épaulement radial délimitant ainsi deux zones de retenue pour deux liens souples.

Il va de soi que cette entretoise peut avoir toute autre forme et recevoir au moins un lien souple.

On dispose ainsi d'un système soit souple, soit rigide soit partiellement souple et partiellement rigide, que ce soit en première intention ou en reprise, sans qu'il y ait à explanter l'implant. Il suffira juste de démonter le dispositif au niveau d'une articulation et de réaliser un nouveau montage.

II - Selon un deuxième mode préférentiel de l'invention, l'entretoise est constituée d'un segment cylindrique destiné à recevoir un lien souple, percé de part en part et comportant en l'une de ses extrémités un fraisage cylindrique perpendiculaire à son axe.

Sur ce fraisage repose la douille cylindrique d'un étau destiné à retenir en ses mâchoires une barre rigide.

Avantageusement, la douille comporte un transpercement central composé de deux cônes communiquant par leur sommet et destiné à être inséré sur l'axe de l'implant.

Ainsi, l'étau dispose d'une liberté de débattement latéral. Il s'ensuit que la barre insérée dans l'étau pourra s'orienter en fonction de la position de l'implant.

Selon une variante de ce deuxième mode préférentiel, cette barre, plutôt de section ronde, est coudée à 90° à chacune de ses extrémités, ces coudes servant de supports à un lien souple. Dans ce cas, l'entretoise décrite ne recevra plus de lien souple.

Plusieurs modes de réalisation du dispositif global conforme à l'invention vont être maintenant décrits plus en détail, mais uniquement à titre d'exemples non limitatifs, en référence aux dessins annexés, dans lesquels :
- la figure 1 illustre la conception générale d'un stabilisateur intervertébral souple mis en place en première intention.
- la figure 2 est une vue de face de ce stabilisateur intervertébral à la suite d'une reprise avec des plaques rigides, représenté dans un exemple de configuration d'implantation, selon le premier mode de réalisation, et la première caractéristique de l'invention,
- la figure 3 représente une vue de face en coupe du stabilisateur intervertébral de la figure 2,
- la figure 3a est une vue latérale de la plaque dont l'axe longitudinal est perpendiculaire à celui de l'implant,
- la figure 3b est une vue latérale de la plaque avec un débattement par rapport à l'implant,
- la figure 3c est une vue en coupe de dessus de la plaque,
- la figure 3d est une vue latérale en coupe de la plaque,
- la figure 4 est une vue de face en coupe des articulations 2-3-4 selon la deuxième caractéristique de l'invention,
- la figure 4a est une vue latérale de la plaque dont l'axe longitudinal est perpendiculaire à celui de l'implant,
- la figure 4b est une vue latérale de la plaque avec un débattement par rapport à l'implant,
- la figure 4e est une vue en coupe de dessus de la plaque,

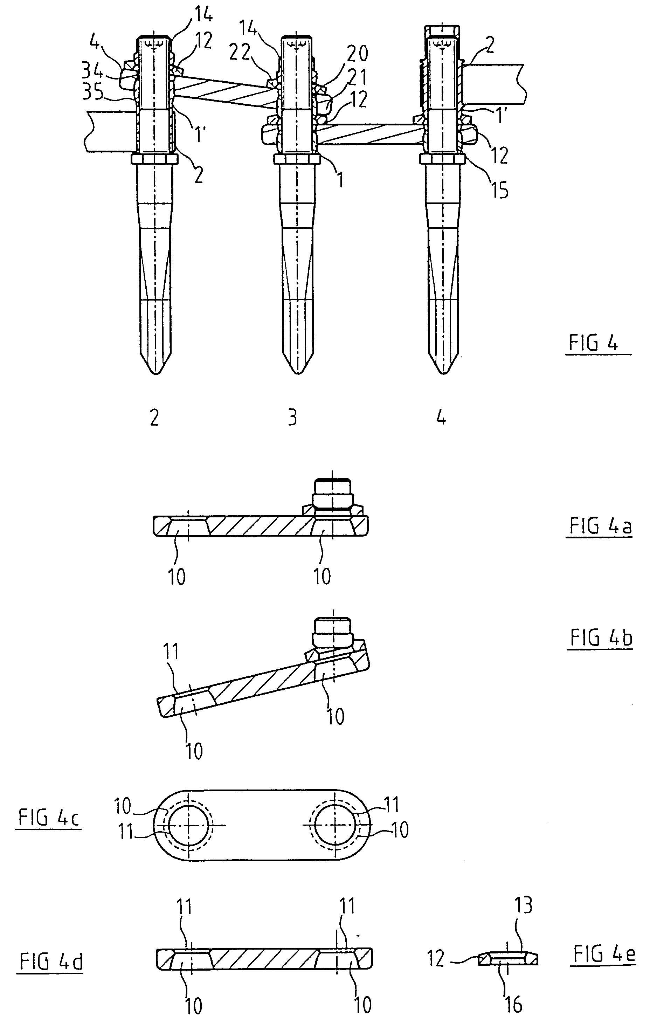

- la figure 4d est une vue latérale en coupe de la plaque,
- la figure 4e illustre une vue en coupe de la rondelle,

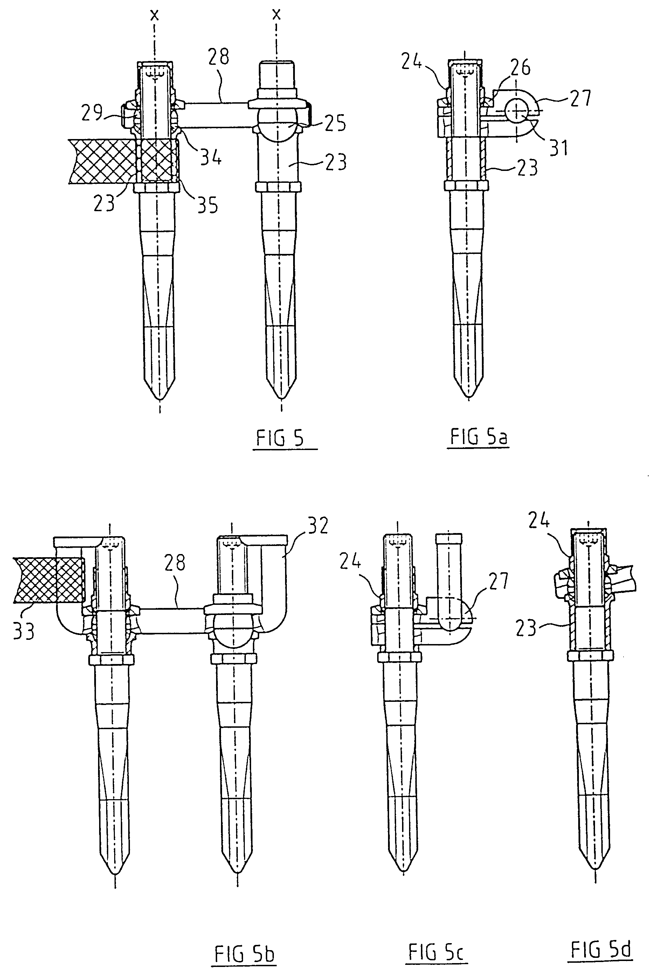

- la figure 5 est une vue de face en coupe de l'articulation 1-2 selon le deuxième mode de réalisation de l'invention,
- la figure 5a est une vue latérale des implants de la figure 5,
- la figure 5b représente la figure 5 selon une variante de l'invention,
- la figure 5c est une vue latérale de la figure 5b,
- la figure 5d est la même vue que la figure 5a avec un débattement par rapport à la plaque,

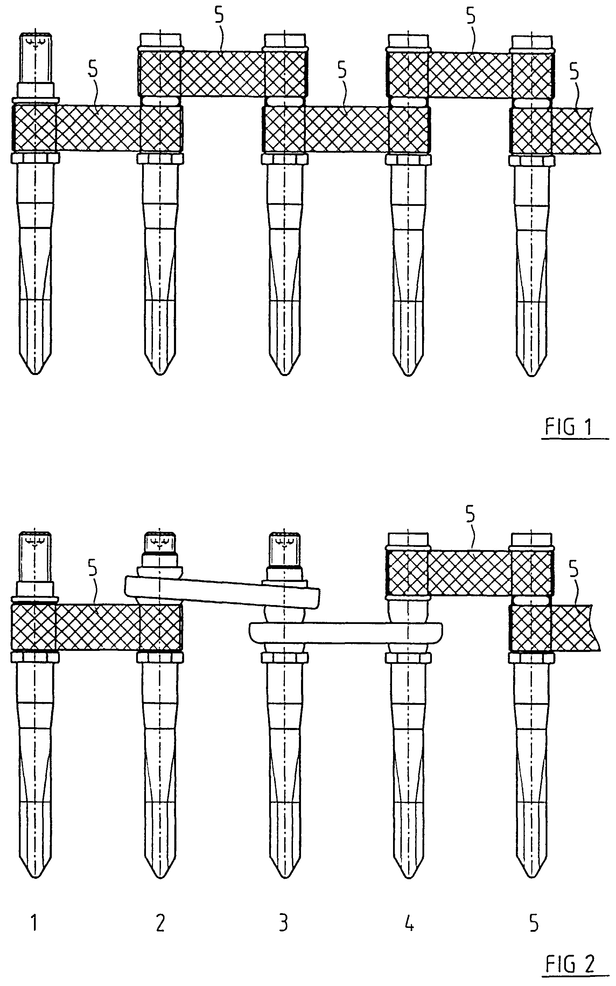

La figure 1 indique un stabilisateur intervertébral composé de plusieurs liens souples montés en chaîne, à l'aide d'implants métalliques, au dos des vertèbres constituant le tronçon du rachis traité.

Comme l'indique la figure 2, les articulations souples 2-3-4 du stabilisateur intervertébral ont été remplacées par des plaques rigides sans qu'il y ait à explanter l'implant.

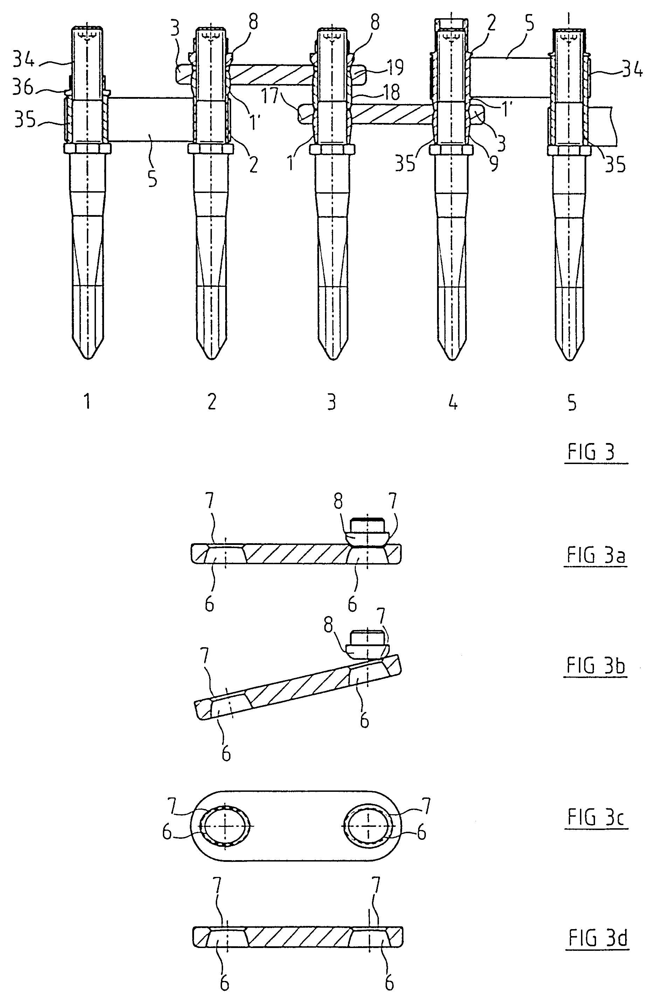

Le stabilisateur représenté sur les figures 3, 4, 5, 5b est réalisé à l'aide d'implants, chacun associé à :
- une entretoise destinée à supporter un ou deux liens souples
- ou une entretoise destinée à supporter une ou deux plaques ou barres rigides
- ou encore une entretoise destinée à supporter un lien souple et une plaque ou barre rigide.

Le premier type d'entretoise est percé de part en part et constitué d'un segment de sphère seul (1). Le troisième type d'entretoise est constitué d'un segment de sphère prolongé (1') d'un segment cylindrique (2). Le segment de sphère étant destiné à recevoir l'extrémité d'une plaque rigide (3, 4), le segment cylindrique étant destiné à recevoir un lien souple (5).

Les implants 2 et 4 de la figure 3 décrivent chacun une entretoise constituée d'un segment de sphère prolongé (1') du segment cylindrique (2). Cette entretoise reçoit une plaque (Fig. 3 à 3d) comportant en chacune de ses extrémités une concavité (6) parfaitement congruente à la convexité du segment de sphère (1'). Cette concavité est suivie d'un perçage débouchant sur l'autre face dans une concavité oblongue (7) suivant l'axe longitudinal de la plaque.

L'implant reçoit avec ce type de plaque un segment de sphère (8, 9) présentant une même convexité sphérique que celle du segment de sphère (1, 1').

Les implants 2 et 4 de la figure 4 décrivent chacun une entretoise constituée d'un segment de sphère prolongé (1') du segment cylindrique (2). Cette entretoise reçoit une plaque (Fig. 4 à 4d) comportant à chacune de ses extrémités, une concavité (10) parfaitement congruente à la convexité du segment de sphère (1'). Cette concavité est suivie d'un perçage débouchant sur l'autre face (11).

L'implant reçoit avec ce type de plaque :
. une rondelle (12) comportant une concavité (13) congruente à la convexité du segment de sphère (14, 15) et suivie d'un perçage débouchant sur l'autre face (16).
. un segment de sphère (14, 15) percé de part en part, et dont la convexité est congruente à la concavité (11) de la rondelle (12).

L'entretoise uniquement constituée d'un segment de sphère (1) reçoit une ou deux plaques :
- Avec le premier type de plaque (3), il suffira d'insérer entre l'extrémité (17) de la plaque et le segment de sphère (8) l'extrémité (19) d'une plaque, le segment de sphère (18), la concavité sphérique (6) de la plaque recevant la convexité sphérique du segment de sphère (18).
- Avec le deuxième type de plaque (4), il suffira d'insérer entre la rondelle (12) et le segment de sphère (14), le segment de sphère (20), l'extrémité (21) d'une plaque et une rondelle (22).

L'entretoise constituée uniquement du segment cylindrique, destiné à recevoir un lien souple ou d'un segment cylindrique, comprenant en son milieu un épaulement radial délimitant ainsi deux zones de retenue pour deux liens souples, est conforme à la tête extra-osseuse des implants décrits dans les brevets FR 89 01445 et 91 01288 (voir fig. 1-2).

Le deuxième type d'entretoise est constitué d'un segment cylindrique (23) destiné à recevoir un lien souple et comportant en l'une de ses extrémités un fraisage cylindrique perpendiculaire à son axe (X).

Sur ce fraisage, repose la douille cylindrique (25) d'un étau (26) destiné à retenir en ses mâchoires (27) une barre rigide (28).

La douille comporte un transpercement central composé de deux cônes (29) communiquant par leur sommet (30) et destiné à être inséré sur l'axe (X) de l'implant, en permettant une certaine angulation.

La barre destinée à être insérée dans l'étau est de section ronde (31) et coudée à 90° (32) à chacune de ses extrémités, ces coudes servant de supports à un lien souple (33).

L'axe comporte un filetage en son extrémité supérieure (34) et est lisse à sa base (35). Chaque entretoise comporte en son perçage un taraudage interne en son extrémité supérieure (36) destiné à recevoir une partie du filetage de l'axe.

Cet axe, fileté uniquement en son extrémité supérieure, permet, d'une part, au chirurgien-orthopédiste de gagner du temps lors du vissage de l'entretoise sur l'implant et, d'autre part, d'éviter le dévissage de la tige intra-osseuse, lors du démontage de l'entretoise quand il s'agira de défaire l'assemblage mis en place au niveau d'une quelconque articulation.

L'axe décrit n'est qu'une forme privilégiée. Il va de soi qu'il peut être fileté complètement, comporter un évasement en sa base. L'axe peut être amovible de la tige intra-osseuse et, dans ce cas, être précédé d'un filetage qui sera destiné à être vissé à l'implant, ce dernier comportant un taraudage interne. Le perçage de l'entretoise sera dans ce cas, bien entendu, adapté à la forme de l'axe.

L'axe peut comporter en son extrémité supérieure le segment de sphère (14, 15) décrit dans le premier type d'entretoise.

Le verrouillage de l'entretoise sur l'implant est effectué grâce au filetage décrit précédemment ou à l'aide d'un écrou de verrouillage (24).

## Revendications

1. Dispositif de stabilisation du rachis à mettre en place entre au moins deux vertèbres consécutives, ledit dispositif comprenant les éléments suivants :
a) au moins deux implants pédiculaires implantables chacun dans une vertèbre respective, chacun desdits implants comportant un axe extra-osseux,
b) un premier type d'entretoise interchangeable constitué d'un segment de sphère seul (1) apte à être associé à l'axe extra-osseux de chaque implant pédiculaire,
c) un deuxième type d'entretoise interchangeable constitué d'un segment cylindrique (23) apte à être associé à l'axe extra-osseux de chaque implant pédiculaire, et
d) un lien souple (5, 33) apte à être reçu par le segment cylindrique (23), ledit dispositif étant caractérisé en ce qu'il comprend par ailleurs
e) un troisième type d'entretoise interchangeable constitué d'un segment de sphère (1') prolongé d'un segment cylindrique (2) apte à être associé à l'axe extra-osseux de chaque implant pédiculaire, et
f) une ou plusieurs plaques rigides (3, 4) comportant au moins à chacune de ses extrémités une concavité sphérique (6) parfaitement congruente à la convexité du segment de sphère seul (1, 8, 9, 14, 15, 18, 20) ou d'un segment de sphère (1') prolongé d'un segment cylindrique suivi d'un perçage (11) débouchant sur l'autre face dans une concavité oblongue suivant l'axe longitudinal de la plaque rigide (3, 4), ladite plaque rigide (3, 4) étant apte à être reçue par les différents types d'entretoises interchangeables.

2. Dispositif global selon la revendication 1, caractérisé en ce que la plaque reçoit, sur son autre face, un segment de sphère (8, 9) parfaitement congruent à la concavité (10) de la plaque.

3. Dispositif global selon la revendication 1, caractérisé en ce que la plaque reçoit une rondelle (12) dont la concavité (13) est parfaitement congruente à la convexité du segment de sphère (14, 15) et suivie d'un perçage débouchant sur l'autre face (16).

4. Dispositif global selon la revendication 1, caractérisé en ce que l'implant est associé par son axe à un deuxième type privilégié d'entretoise constituée d'un segment cylindrique (23) destiné à recevoir un lien souple, percée de part en part et comportant en l'une de ses extrémités un fraisage cylindrique perpendiculaire à son axe (X) sur lequel repose la douille cylindrique (25) d'un étau (26) retenant en ses mâchoires (27) une barre rigide (28) ; ladite douille comporte un transpercement central composé de deux cônes (29) communiquant par leur sommet (30), et destiné à être inséré sur l'axe (X) de l'implant.

5. Dispositif global selon la revendication 4, caractérisé en ce que la barre insérée dans l'étau est de section ronde (31) et coudée à 90° (32), à chacune de ses extrémités, ces coudes servant de supports à un lien souple (33).

6. Dispositif global selon la revendication 3, caractérisé en ce que l'entretoise constituée d'un segment de sphère reçoit :
- un premier type de plaque (3) en insérant entre son extrémité (17) et le segment de sphère (8), l'extrémité (19) de la plaque, le segment de sphère (18), la concavité sphérique (6) de la plaque recevant la convexité sphérique dudit segment de sphère (18) ;
- un deuxième type de plaque (4) en insérant entre la rondelle (12) et le segment de sphère (14), le segment de sphère (20), l'extrémité (21) de la plaque et la rondelle (22).

7. Dispositif global selon la revendication 1, caractérisé en ce que l'axe extra-osseux, lisse à sa base (35), est fileté en son extrémité supérieure (34) ; chaque entretoise comporte en son perçage un taraudage interne en son extrémité supérieure (36) destiné à recevoir une partie du filetage de l'axe.

8. Dispositif global selon la revendication 7, caractérisé en ce que l'axe extra-osseux comporte en son extrémité supérieure un écrou de verrouillage (24) de l'entretoise à l'implant.

## Claims

1. Spinal stabilization device, to be placed between at least two consecutive vertebrae, the aforesaid device including the following elements:
a) at least two pedicle implants which can each be implanted in a respective vertebra, each of the aforesaid implants consisting of an extra-osseous pin axis;
b) a first type of interchangeable brace constituted of a sphere segment alone (1) capable of being associated with the extra-osseous pin axis of each pedicle implant;
c) a second type of interchangeable brace constituted of a cylindrical segment (23) capable of being associated with the extra-osseous pin axis of each pedicle implant, and
d) a supple link (5, 33) capable of being received by the cylindrical segment (23), the aforesaid device being characterized by the fact that it also includes:
e) a third type of interchangeable brace constituted of a sphere segment (1') prolonged by a cylindrical segment (2) capable of being associated with the extra-osseous pin axis of each pedicle implant, and
f) one or several rigid plates (3, 4) consisting, at least at each of its ends, a spherical concavity (6), perfectly congruent with the convexity of the sphere segment alone (1, 8, 9, 14,15, 18, 20) or of a sphere segment (1') which is prolonged by a cylindrical segment followed by a piercing (11) through to the other side in an oblong concavity (7) along the longitudinal axis of the rigid plate (3, 4), the aforesaid rigid plate (3, 4) being capable of being received by the different types of interchangeable braces.

2. Global device according to claim 1, characterized by the fact that the plate receives a sphere segment (8, 9) on its other side, perfectly congruent with the concavity (10) of the plate.

3. Global device according to claim 1, characterized by the fact that the plate receives a washer (12), the concavity (13) of which is perfectly congruent with the convexity of the sphere segment (14, 15) and is followed by a piercing (16) through to the other side.

4. Global device according to claim 1, characterized by the fact that the implant is associated by means of its axis with a second privileged type of brace, made up of a cylindrical segment (23) intended to receive a supple link, pierced right through and having, at one of its ends, a cylindrical milling perpendicular to its axis (X) on which the cylindrical sleeve (25) of a vice (26) intended to retain a rigid bar (28) in its jaws (27) is supported; the aforesaid sleeve has a central transpiercing made up of two cones (29) communicating through their vertex (30) and intended to be inserted on the axis (X) of the implant.

5. Global device according to claim 4, characterized by the fact that the bar inserted in the vice has round cross-section (31) and is elbowed at 90° (32) at each of its ends, these elbows serving as supports for a supple link (33).

6. Global device according to claim 3, characterized by the fact that the brace constituted by a sphere segment receives:
- a first type of plate (3) by inserting between its end (17) and the sphere segment (8) the end (19) of the plate, the sphere segment (18), the spherical concavity (6) of the plate receiving the spherical convexity of the said sphere segment (18);
- a second type of plate (4) by inserting between the washer (12) and the sphere segment (14) the sphere segment (20), the end (21) of the plate and the washer (22).

7. Global device according to claim 1, characterized by the fact that the extra-osseous pin axis has threading on its upper end (34) and is smooth at its base (35); each brace having internal femal threading in the piercing at its upper end (36) intended to receive a part of the thread of the pin.

8. Global device according to claim 7, characterized by the fact that the extra-osseous pin axis includes, at its upper end, locking of the brace (24) onto the implant.

## Patentansprüche

1. Vorrichtung zur Stabilisierung der Wirbelsäule, die zwischen mindestens zwei aufeinanderfolgenden Wirbeln eingesetzt wird, wobei die besagte Vorrichtung die folgenden Elemente umfaßt:
a) mindestens zwei Stielimplantate, die jeweils in den entsprechenden Wirbeln implantiert werden, wobei jedes der besagten Implantate über einen Stift verfügt, der sich außerhalb des Knochens befindet,
b) einen auswechselbaren Querriegel erster Art, bestehend aus einem einzelnen Sphärensegment (1), der mit dem außerhalb des Knochens liegenden Stiftes eines jeden Stiel-implantats verbunden werden kann,
c) einen auswechselbaren Querriegel zweiter Art, bestehend aus einen einzelnen Zylindersegment (23), der mit dem außerhalb des Knochens liegenden Stiftes eines jeden Stielimplantats verbunden werden kann, und
d) ein biegsames Band (5, 33), das vom Zylindersegment aufgenommen werden kann (23) die besagte Vorrichtung ist dadurch gekennzeichnet, daß sie außerdem folgendes Umfaßt
e) einen auswechselbaren Querriegel dritter Art, bestehend aus einem Sphärensegment (1'), welches durch ein Zylindersegment (2) verlängert wird, das mit dem außerhalb des Knochens liegenden Stiftes eines jeden Stielimplantats verbunden werden kann? und
f) eine oder mehrere starre Platten (3, 4), welche mindestens an jedem Ende eine kugelförmige Konkavität (6) aufweist bzw. aufweisen, die perfekt mit der konvexen Fläche des einzelnen Sphärensegment (1, 8, 9, 14, 15, 18, 20) oder mit einem durch ein zylindrisches Segment verlängertes Sphärensegment (1') übereinstimmt bzw. übereinstimmen, gefolgt von einer Bohrung (11), die auf der anderen Fläche in einer nach der Längsachse der Platte gerichteten länglichen konkaven Kuhle (7) mündet, wobei die besagte starre Platte (3, 4) von den verschiedenen Typen auswechselbarer Querriegel aufgenommen werden kann.

2. Globalvorrichtung gemäß Anspruch 1, dadurch gekennzeichnet, daß die Platte auf der anderen Fläche ein zweites Sphärensegment (8, 9) aufnimmt, das perfekt mit der Konkavität (10) der Platte übereinstimmt.

3. Globalvorrichtung gemäß Anspruch 1, dadurch gekennzeichnet, daß die Platte eine Scheibe (12) aufnimmt, deren konkave Fläche (13) perfekt mit der konvexen Fläche des Sphärensegments (14, 15) übereinstimmt, gefolgt von einer Bohrung(16), die auf der anderen Seite mündet.

4. Globalvorrichtung gemäß Anspruch 1, dadurch gekennzeichnet, daß das Implantat über seine Achse mit einer zweiten Art eines bevorzugten Querriegels verbunden ist, bestehend aus einem zylindrischen Segment (23), welcher ein biegsames Band aufnimmt und an einem Ende eine senkrecht zur Längsachse (X) stehendes zylindrisch gefrästes Teil aufweist, auf der die zylindrische Buchse (25) einer Schraubklemme (26) ruht, die eine starre Stange (28) zwischen den Backen (27) festhält ; wobei die Buchse in ihrer Mitte eine Durchbohrung aufweist, bestehend aus zwei an der oberen Spitze (30) zusammengefügten Kegeln (29), welche auf die Achse (X) des Implantats aufgesteckt wird.

5. Globalvorrichtung gemäß Anspruch 4, dadurch gekennzeichnet, daß die in die Schraubklemme eingeführte Stange einen runden Querschnitt (31) hat und an jedem Ende 90° ellenbogenförmige Biegungen (32) aufweist, wobei diese Biegungen als Stütze für ein biegsames Band (33) dienen.

6. Globalvorrichtung gemäß Anspruch 3, dadurch gekennzeichnet, daß der Querriegel, welcher aus einem Sphärensegment besteht, folgendes aufnimmt:
- eine erste Art von Platte (3), durch Einfügen des Endes (19) der Platte und des Sphärensegments (18) zwischen deren Ende (17) und dem Sphärensegment (8), wobei die kugelförmige Konkavität (6) der Platte die kugelförmige Konvexität des Sphärensegments (18) aufnimmt,
- eine zweite Art von Platte (4), durch Einfügen des Sphärensegments (20), des Endes (21) der Platte und der Scheibe (22) zwischen der Scheibe (12) und dem Sphärensegment (14).

7. Globalvorrichtung gemäß Anspruch 1, dadurch gekennzeichnet, daß der Stift, der sich außerhalb des Knochens befindet, unten glatt ist (35) und am oberen Ende ein Gewinde (34) aufweist und, wobei die Durchbohrung eines jeden Querriegels am oberen Ende ein Innengewinde (36) aufweist, um einen Teil des Stiftgewindes aufzunehmen.

8. Globalvorrichtung gemäß Anspruch 7, dadurch gekennzeichnet, daß der außerhalb des Knochens liegende Stift am oberen Ende über eine Mutter (24) verfügt, um den Querriegel mit dem Implantat zu verriegeln.
